# EUROPÄISCHE PATENTSCHRIFT

(11) **EP 2 849 826 B1**
(45) Veröffentlichungstag und Bekanntmachung des Hinweises auf die Patenterteilung: **13.01.2016**
(21) Anmeldenummer: 13724507.2
(22) Anmeldetag: 14.05.2013
(51) Int. Cl.: A61M 5/34, A61M 5/31, B29C 45/00, B29C 45/03, B29C 45/14, B29C 45/26, B29C 45/40

(54) **VERFAHREN ZUR HERSTELLUNG EINES SPRITZENZYLINDERS FÜR MEDIZINISCHE ZWECKE SOWIE EINE VORRICHTUNG ZUR DURCHFÜHRUNG DES VERFAHRENS**
METHOD OF PRODUCING A SYRINGE BARREL FOR MEDICAL PURPOSES AND DEVICE FOR CARRYING OUT SAID METHOD
PROCÉDÉ DE FABRICATION D'UN CORPS DE SERINGUE À USAGE MÉDICAL ET DISPOSITIF SERVANT À RÉALISER CE PROCÉDÉ

(30) Priorität: 14.05.2012 CH 676122012
(43) Veröffentlichungstag der Anmeldung: 25.03.2015
(73) Patentinhaber: Schöttli AG, 8253 Diessenhofen (CH)
(72) Erfinder: ZAHN, Lothar, 78262 Gailingen (DE)
(74) Vertreter: BOVARD AG
(86) Internationale Anmeldenummer: PCT/CH2013/000082
(87) Internationale Veröffentlichungsnummer: WO 2013/170393

(56) Entgegenhaltungen:
- EP-A1- 2 140 896
- WO-A1-2012/043544
- US-A- 3 330 004
- US-A1- 2012 010 573

## Beschreibung

Gegenstand der Erfindung ist ein Verfahren zur Herstellung eines Spritzenzylinders gemäss Oberbegriff des Patentanspruchs 1. Die Erfindung betrifft weiter eine Vorrichtung zur Durchführung des Verfahrens gemäss Oberbegriff des Patentanspruchs 5.

Spritzen für medizinische Zwecke umfassen einen Spritzenzylinder, an dessen vorderen Ende eine Nadel angeordnet ist sowie ein Kolben, mit dem einerseits das zu spritzende Medium aufgezogen und später injiziert wird. In den häufigsten Fällen wird die Nadel unabhängig von der Spritze beziehungsweise vom Spritzenzylinder und -kolben verpackt geliefert. Es ist aber auch bekannt, insbesondere für Spritzen zum Injizieren von Impfmitteln und dergleichen, diese mit bereits aufgesetzter Nadel herzustellen. Bei der Herstellung solcher Spritzen wird die am vorderen Ende zugespitzte Nadel bei geöffnetem Werkzeug mit einem Handlinggerät durch die Kavität im Spritzgiesswerkzeug hindurch eingeführt und danach beim Spritzvorgang durch den flüssigen Kunststoff teilweise umschlossen. Zum Einsetzen der Nadel durch das geöffnete Werkzeug ist es notwendig, die Nadeln in geeigneter Weise zu konfektionieren, damit sie vom Handlinggerät erfasst und dann zwischen dem geöffneten Werkzeug in die Kavität eingeführt werden kann. Dies führt zu einer Verkleinerung der Zyklen pro Zeiteinheit und folglich zu höheren Herstellungskosten.

Aus der WO2012/003221 A1 ist weiter eine Medizinalspritze mit integrierter Nadel bekannt. Die Nadel wird mit Klemmbacken während des Spritzvorgangs derart positioniert gehalten, dass das dem geschliffenen Ende der Nadel gegenüberliegende Ende die innere Wandung des Spritzenzylinders sicher nicht überragt. Vorzugsweise wird das innenliegende Ende der Nadel sogar etwas zurückversetzt gehalten, damit auch bei einem allfälligen Schrumpf des Kunststoffs des Spritzenzylinders das Ende nicht in den Innenraum ragt und somit das vollständige Auspressen der Injektionsflüssigkeit nicht behindert. Im Weiteren wird die Bohrung der Nadel während des Spritzvorgangs durch einen Faden oder Draht (Strand) verschlossen gehalten, um das Eindringen von Kunststoff in die Nadel zu verhindern. Die Fertigung eines solchen Spritzenzylinders mit eingesetzter Nadel ist sehr kostenaufwändig. In EP 2140896 A1 wird auch ein Verfahren zur Herstellung eines mit einer Nadel ausgestatteten Spritzenzylinder beschrieben. Dabei wird die Nadel durch eine für den Spitzenzylinder vorgesehene Kavität in den distalen Teil des Spritzwerkzeugs eingesetzt. Während des Spritzvorgangs wird die Nadel mittels eines elastischen Materials bzw. einer Feder gegen einen Kern gedrückt. Das Zuführen der Nadel zum Spritzwerkzeug in diesem Verfahren dauert allerdings relativ lange.

Eine Aufgabe der vorliegenden Erfindung besteht nun darin, die Zuführung der Nadel zum Spritzgiesswerkzeug dahingehend zu optimieren, damit die Zeitspanne, in der das feststehende und das bewegliche Werkzeugteil auseinandergefahren sind, verkleinert werden kann.

Gelöst wird diese Aufgabe durch ein Verfahren gemäss den Merkmalen des Patentanspruchs 1 und durch eine Vorrichtung gemäss den Merkmalen des Patentanspruchs 5.

Die Verlagerung des Zuführorts auf die Rückseite des feststehenden Werkzeugteils ermöglicht es, die beiden Werkzeugteile der Spritzgiessform unmittelbar nach dem Ausstossen des oder der frisch gespritzten Spritzlinge, hier Spritzenzylinder, samt eingesetzter Nadel, sofort wieder zu schliessen und gleichzeitig die nächste Nadel so weit in die Kavität einzubringen, damit deren hinteres Ende umspritzt werden kann. Das hintere Ende der Nadel wird dabei in eine Präzisionsbohrung im Kern des Spritzenzylinders eingefahren und dadurch die Bohrung in der Nadel dicht verschlossen, so dass kein Kunststoff eindringen kann. Das Einführen der Nadel in die Kavität für den Spritzenzylinder und die Bohrung im Kern von ausserhalb des Werkzeugs durch das feststehende Werkzeug hindurch hat weiter den Vorteil, dass die Nadel nicht mit dem spitzen Ende voran, sondern mit dem stumpfen rückwärtigen Ende eingeschoben und das spitze geschliffene Ende während des Einschiebens nicht verletzt werden kann. Ein weiterer Vorteil besteht darin, dass die Vereinzelung der Nadeln auf einfache Weise erfolgen kann und keine komplizierten Handlingsysteme und auch keine Konfektionierung der Nadeln notwendig sind.

Anhand eines illustrierten Ausführungsbeispiels wird die Erfindung näher erläutert. Es zeigen:

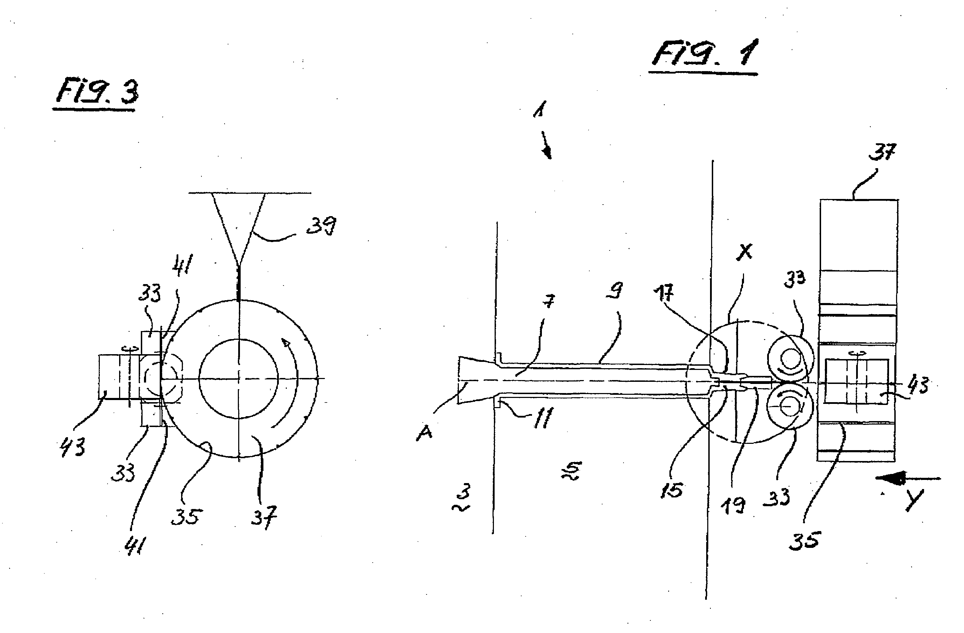

Figur 1 Schematisch einen Vertikalschnitt durch die beiden Formhälften sowie eine Vorrichtung zum Zu- und Einführen der Nadeln,

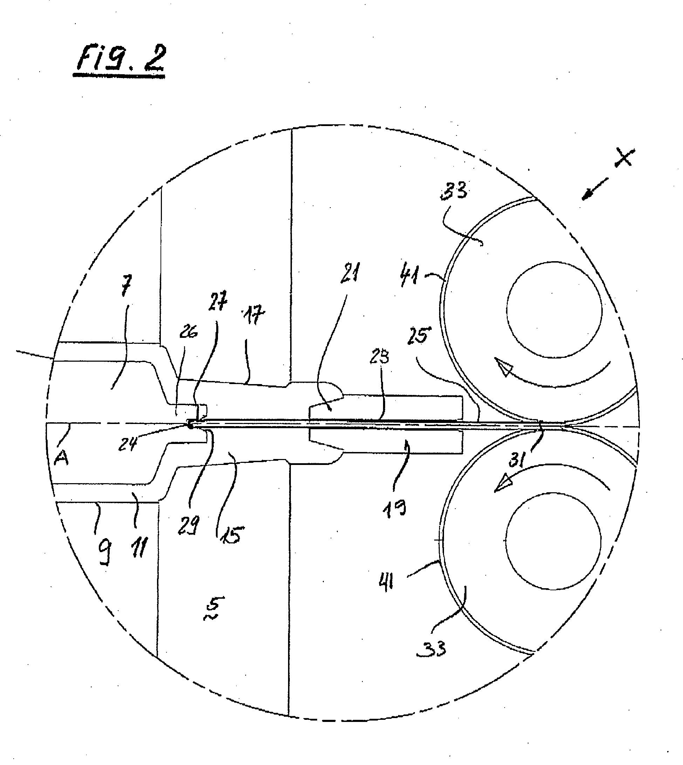

Figur 2 eine vergrösserte Darstellung des Bereichs X in Figur 1 und
Figur 3 eine Ansicht der Vereinzelung- und Zuführvörrichtung aus Richtung des Pfeils Y in Figur 1.

Das Bezugszeichen 3 stellt schematisch den beweglichen Teil eines Spritzgiesswerkzeugs 1 und das Bezugszeichen 5 den feststehenden Teil eines Spritzgiesswerkzeugs 1 dar. Am beweglichen Teil 3 des Spritzgiesswerkzeugs 1 ist ein Kern 7 befestigt, welcher in die Kavität 9 für einen Spritzenzylinder 11 im feststehenden Werkzeugteil 5 hineinragt. Anschliessend an die zylindrische Kavität 9 ist auf der rechten Seite in den Figuren ein beispielsweise konisch verlaufender, die Nadelaufnahme 15 bildender Kavitätsabschnitt 17 ersichtlich. In das Ende des Kavitätsabschnitts 17 ragt ein Nadelführungsrohr 19 mit einem kegelstumpfförmigen Ende 21. Eine axiale Bohrung 23 im Führungsrohr 19 weist einen Durchmesser auf, der das hintere Ende 24 einer Nadel 25 im Wesentlichen spielfrei und dichtend während des Spritzens führen kann. Am freien Ende 26 des Kerns 7 ist ein Sackloch 27 ausgebildet, das eingangsseitig vorzugsweise einen konischen Einlaufbereich 29 umfasst.

Das vordere mit einer Spitze versehene Ende 31 der Nadel 25 wird von zwei Transportrollen 33 gehalten. Auf der Peripherie einer Zuführtrommel 37 sind beabstandet eine Mehrzahl von Nuten 35 eingelassen, in welche von einem Zuführtrichter 39 oder einer anderen geeigneten Vereinzelungsvorrichtung einzelne Nadeln 25 den Transportrollen 33 zugeführt werden können.

Das Überführen der Nadeln 17 aus den Nuten 35 in die Zuführtrommel 37 zwischen die beiden Transportrollen 33, beziehungsweise in auf deren Peripherie angebrachte Rillen 41, erfolgt über eine Reibwalze 43, welche mit ihrer gummierten Oberfläche die Nadeln 25 axial aus den Nuten 35 auf der Zuführtrommel 37 hinausführt.

Nachfolgend werden das Verfahren und die Vorrichtung kurz erläutert. Nach dem Öffnen der beiden Spritzgiessformhälften 3,5 durch Wegfahren des beweglichen Teil 3 vom feststehenden Teil 5 und Ausstossen eines fertiggestellten und mit eingespritzter Nadel 17 versehenen Spritzenzylinders 11 wird das Spritzgiesswerkzeug 1 sofort wieder geschlossen, das heisst der bewegliche Formteil 3 wieder an den feststehenden Formteil 5 herangeführt. Gleichzeitig, nachträglich oder kurz vorher wird von der Reibwalze 43 eine Nadel 25 aus einer Nut 35 an der Zuführtrommel 37 zwischen die Transportrollen 33 beziehungsweise in die Rillen 41 an den Transportrollen 33 eingeschoben und von den drehenden Transportrollen 33 in axialer Richtung A durch die Bohrung 23 im Führungsrohr 19 hindurch in das Sackloch 27 am vorderen Ende des Kerns 7 bis zum Anschlag vorgeschoben. Die Nadel 25 wird von den Transportrollen 33 im Sackloch 27 axial angepresst gehalten, bis die Kavität 9, in welcher der Spritzenzylinder 11 entsteht, vollständig mit Kunststoff aufgefüllt ist. Das Festhalten der Nadel 25 während des Einspritzens des Kunststoffs kann - wie beschrieben - durch das Drehmoment der Transportrollen 33 erfolgen oder auch durch eine andere, die Nadel 25 im Sackloch 27 anpressend festhaltende Vorrichtung (keine Abbildung), falls die Beschickung des Spritzgiesswerkzeugs 1 durch eine andere Vorrichtung als die hier beschriebene erfolgt.

Nach Beendigung des Einspritzvorgangs und der notwendigen Kristallisations- und Abkühlzeit für den Kunststoff kann der Spritzenzylinder 11 mit der nun angespritzten Nadel 25 durch Öffnen der beiden Formteile 3 und 5 ausgeworfen und danach sofort wieder geschlossen zu werden, während nach dem Ausstossen des Spritzenzylinders 11 bereits eine neue Nadel 25 durch das Führungsrohr 19 in den feststehenden Teil 5 in die Kavität 9 für den Spritzenzylinder 11 eingeführt wird.

Die Vorrichtung für das Zuführen der Nadeln 25 weist einen sehr einfachen Aufbau auf, da die Nadeln 25 vom Zuführtrichter 39 z.B. durch Schwerkraft in die Nuten 35 auf der Zuführtrommel 37 fallen und dann durch die Reibwalze 43 den Transportrollen 33 zugeführt werden können. Das Positionieren der Nadel 25 erfolgt zwangsweise mit dem Führungsrohr 19 und das Abdichten der Bohrung in der Nadel durch das Sackloch27, welches das Ende der Nadel 25 dichtend umschliesst.

## Patentansprüche

1. Verfahren zur Herstellung eines Spritzenzylinders (11) mit eingesetzter Nadel (25) für medizinische Zwecke, bei dem die Nadel (25) in das Spritzgiesswerkzeug (1) vor dem Einspritzen des Kunststoffs eingesetzt und anschliessend vom Kunststoff teilweise umspritzt wird,
**dadurch gekennzeichnet**,
die Nadel (25) während des Ausstossens eines Spritzenzylinders (11) samt darin eingespritzter Nadel (25) oder danach von ausserhalb des Spritzgiesswerkzeugs (1) von hinten durch den feststehenden Teil (5) des Spritzgiesswerkzeugs (1) hindurch mit ihrem hinteren Nadelende in eine Kavität (9) eingeschoben und an ihrem vorderen Ende im Bereich der Nadelspitze während des Spritzzyklus' durch eine in Einschubrichtung wirkende Kraft gehalten wird.

2. Verfahren nach Anspruch 1, **dadurch gekennzeichnet, dass** die Nadel (25) in das bereits geschlossene Werkzeug eingeführt wird.

3. Verfahren nach einem der Ansprüche 1 oder 2, **dadurch gekennzeichnet, dass** das hintere Ende der Nadel (25) in ein Sackloch (27) am vorderen Ende des Kerns (7), der am beweglichen Teil (3) des Spritzgiesswerkzeugs (1) gehalten ist, eingeführt und von einem die Nadel (25) einschiebenden Transportmittel (33) festgehalten wird.

4. Verfahren nach Anspruch 3, **dadurch gekennzeichnet, dass** das Transportmittel (33) die Nadel (25) am Ende des Spritzzyklus' aus der Halte- und Klemmposition freigibt und/oder die Nadel (25) beim Ausstossen aus dem feststehenden Teil (5) weiter vorschiebt.

5. Vorrichtung zur Durchführung des Verfahrens nach Patentanspruch 1, **dadurch gekennzeichnet, dass** hinter dem feststehenden Teil (5) des Spritzgiesswerkzeugs (1) ein Transportmittel (33) zum Einschieben von einzelnen Nadeln (25) durch ein Führungsrohr (19) im feststehenden Teil (5) in die Kavität (9) angeordnet ist.

6. Vorrichtung nach Anspruch 5, **dadurch gekennzeichnet, dass** am freien Ende (26) des Kerns (7), der am beweglichen Teil (3) angeordnet ist, ein Sackloch (27) zur Führung und Aufnahme des hinteren Endes (24) der Nadel (25) ausgebildet ist.

7. Vorrichtung nach Anspruch 6, **dadurch gekennzeichnet, dass** das Sackloch (27) derart dimensioniert ist, dass das hintere Ende (24) der Nadel (25) dicht umschlossen wird.

## Claims

1. Method of producing a syringe barrel (11) with inserted needle (25) for medical purposes, in which the needle (25) is inserted into the injection moulding tool (1) before injection of the plastic and is then partially moulded over by the plastic,
**characterised in that**,
during the ejection of a syringe barrel (11) together with needle (25) moulded-in therein, or subsequently, the needle (25) is inserted, with its rear needle end, into a cavity (9), from outside of the injection moulding tool (1), from the rear, through the fixed part (5) of the injection moulding tool (1), and is held at its front end in the region of the needle tip during the injection cycle through a force acting in insertion direction.

2. Method according to claim 1, **characterised in that** the needle (25) is introduced into the already closed tool.

3. Method according to one of the claims 1 or 2, **characterised in that** the rear end of the needle (25) is introduced into a blind hole (27) on the front end of the core (7), which is held on the movable part (3) of the injection moulding tool (1), and is firmly held by a transport means (33) inserting the needle (25).

4. Method according to claim 3, **characterised in that** the transport means (33) releases the needle (25) from the holding and clamping position at the end of the injection cycle and/or further advances the needle (25) out of the fixed part (5) during the ejection.

5. Device for carrying out the method according to claim 1, **characterised in that** disposed behind the fixed part (5) of the injection moulding tool (1) is a transport means (33) for insertion of individual needles (25) through a guide tube (19) in the fixed part (5) into the cavity (9).

6. Device according to claim 5, **characterised in that** on the free end (26) of the core (7), which is disposed on the movable part (3), a blind hole (27) is formed for guiding and receiving of the rear end (24) of the needle (25).

7. Device according to claim 6, **characterised in that** the blind hole (27) is dimensioned in such a way that the rear end (24) of the needle (25) is surrounded in a tight way.

## Revendications

1. Procédé de fabrication d'un corps de seringue (11) à usage médical comprenant une aiguille insérée (25), lors duquel l'aiguille (25) est insérée dans le dispositif de moulage par injection (1) avant l'injection du plastique et est ensuite partiellement surmoulée par le plastique,
**caractérisé en ce que**
pendant l'opération de démoulage du corps de seringue (11) incluant l'aiguille (25) qui y a été moulée, ou ultérieurement, l'aiguille (25) est insérée à l'aide de son extrémité arrière dans une cavité (9) depuis l'extérieur du dispositif de moulage par injection (1), par l'arrière à travers la partie fixe (5) du dispositif de moulage par injection (1), et est tenue à son extrémité avant au niveau de la pointe par une force agissant dans la direction d'insertion durant le cycle d'injection.

2. Procédé selon la revendication 1, **caractérisé en ce que** l'aiguille (25) est introduite dans le dispositif déjà fermé.

3. Procédé selon l'une quelconque des revendications 1 ou 2, **caractérisé en ce que** l'extrémité arrière de l'aiguille (25) est introduite dans un trou borgne (27) disposé à l'extrémité avant du noyau (7), qui est maintenu à la partie mobile (3) du dispositif de moulage par injection (1), et est y maintenue par un moyen de transport (33) insérant l'aiguille (25).

4. Procédé selon la revendication 3, **caractérisé en ce que** le moyen de transport (33) libère l'aiguille (25) de sa position de maintien et de blocage à la fin du cycle d'injection et/ou continue à faire avancer l'aiguille (25) hors de la partie fixe (5) lors de l'opération de démoulage.

5. Dispositif pour l'exécution du procédé selon la revendication 1, **caractérisé en ce que** derrière la partie fixe (5) du dispositif de moulage par injection (1) est agencé un moyen de transport (33) pour insérer des aiguilles (25) une par une dans la cavité (9) à travers un tube de guidage (19) de la partie fixe (5).

6. Dispositif selon la revendication 5, **caractérisé en ce qu'**à l'extrémité libre (26) du noyau (7), qui est agencé au niveau de la partie mobile (3), est formé un trou borgne (27) pour guider et recevoir l'extrémité arrière (24) de l'aiguille (25).

7. Dispositif selon la revendication 6, **caractérisé en ce que** le trou borgne (27) est dimensionné de telle manière que l'extrémité arrière (24) de l'aiguille (25) y est enserrée de manière étanche.
